# EUROPEAN PATENT SPECIFICATION

(11) **EP 2 988 737 B1**
(45) Date of publication and mention of the grant of the patent: **07.04.2021**
(21) Application number: 14788463.9
(22) Date of filing: 23.04.2014
(51) Int. Cl.: A61K 31/045, A61K 31/46, A61P 25/30, A61K 38/44

(54) **METHODS AND COMPOSITIONS FOR TREATING DISEASES**
VERFAHREN UND ZUSAMMENSETZUNGEN ZUR BEHANDLUNG VON KRANKHEITEN
PROCÉDÉS ET COMPOSITIONS POUR LE TRAITEMENT DE MALADIES

(30) Priority: 23.04.2013 US 201361815206 P
(43) Date of publication of application: 02.03.2016
(73) Proprietor: Nizyme, Inc., San Francisco, CA 94131 (US)
(72) Inventor: KATSNELSON, Ilana, San Francisco, CA 94131 (US)
(74) Representative: Heinze, Ekkehard
(86) International application number: PCT/US2014/035093
(87) International publication number: WO 2014/176309

(56) References cited:
- CN-A- 101 284 128
- CN-A- 101 649 313
- US-A- 5 855 881
- US-A- 5 855 881
- US-A1- 2011 196 134
- LINA YAO ET AL: "Inhibition of aldehyde dehydrogenase-2 suppresses cocaine seeking by generating THP, a cocaine use-dependent inhibitor of dopamine synthesis", NATURE MEDICINE., vol. 16, no. 9, 22 August 2010 (2010-08-22), pages 1024-1028, XP055322905, US ISSN: 1078-8956, DOI: 10.1038/nm.2200
- H. MA ET AL: "Aldehyde dehydrogenase 2 (ALDH2) rescues myocardial ischaemia/reperfusion injury: role of autophagy paradox and toxic aldehyde", EUROPEAN HEART JOURNAL, vol. 32, no. 8, 12 August 2010 (2010-08-12), pages 1025-1038, XP055322907, GB ISSN: 0195-668X, DOI: 10.1093/eurheartj/ehq253
- ISSE, T. ET AL.: 'Aldehyde Dehydrogenase 2 Gene Targeting Mouse Lacking Enzyme Activity Shows High Acetaldehyde Level in Blood, Brain, and Liver after Ethanol Gavages' ALCOHOLISM: CLINICAL AND EXPERIMENTAL RESEARCH vol. 29, no. 11, 2005, pages 1959 - 1964, XP055294032
- ISRAEL , Y. ET AL.: 'Combined Effects of Aldehyde Dehydrogenase Variants and Maternal Mitochondrial Genes on Alcohol Consumption' ALCOHOL RESEARCH AND HEALTH vol. 29, no. 4, 2006, pages 281 - 285, XP055294039
- MA HENG ET AL: "Aldehyde Dehydrogenase 2 Ameliorates Acute Cardiac Toxicity of Ethanol Role of Protein Phosphatase and Forkhead Transcription Factor", JOURNAL OF THE AMERICAN COLLEGE OF CARDIOLOGY, vol. 54, no. 23, 2009, pages 2187-2196, XP029647781, ISSN: 0735-1097, DOI: 10.1016/J.JACC.2009.04.100
- REN J ET AL: "Aldehyde dehydrogenase-2 transgene ameliorates chronic alcohol ingestion-induced apoptosis in cerebral cortex", TOXICOLOGY LETTERS, ELSEVIER BIOMEDICAL PRESS, AMSTERDAM, NL, vol. 187, no. 3, 22 June 2009 (2009-06-22) , pages 149-156, XP026050318, ISSN: 0378-4274, DOI: 10.1016/J.TOXLET.2009.02.019 [retrieved on 2009-03-09]
- HARRY P ET AL: "Disulfiram (antabuse)-ethanol reaction treated by 4-methylpyrazole (4-MP). Two case reports", TOXICOLOGY LETTERS, ELSEVIER BIOMEDICAL PRESS, AMSTERDAM, NL, vol. 95, 1 July 1998 (1998-07-01), page 84, XP027436422, ISSN: 0378-4274, DOI: 10.1016/S0378-4274(98)80333-0 [retrieved on 1998-07-01]
- LI XUE ET AL: "Acetylation-dependent regulation of mitochondrial ALDH2 activation by SIRT3 mediates acute ethanol-induced eNOS activation", FEBS LETTERS, ELSEVIER, AMSTERDAM, NL, vol. 586, no. 2, 28 November 2011 (2011-11-28), pages 137-142, XP028439974, ISSN: 0014-5793, DOI: 10.1016/J.FEBSLET.2011.11.031 [retrieved on 2011-12-07]
- CHEN ET AL: "Activation of aldehyde dehydrogenase-2 reduces ischemic damage to the heart", SCIENCE, AMERICAN ASSOCIATION FOR THE ADVANCEMENT OF SCIENCE, vol. 321, 12 September 2008 (2008-09-12), pages 1493-1495, XP008139634, ISSN: 0036-8075, DOI: 10.1126/SCIENCE.1158554
- WANG G W ET AL: "Acrolein consumption exacerbates myocardial ischemic injury and blocks nitric oxide-induced PKC@e signaling and cardioprotection", JOURNAL OF MOLECULAR AND CELLULAR CARDIOLOGY, ACADEMIC PRESS, GB, vol. 44, no. 6, 1 June 2008 (2008-06-01), pages 1016-1022, XP022708307, ISSN: 0022-2828, DOI: 10.1016/J.YJMCC.2008.03.020 [retrieved on 2008-04-08]
- UMULIS D M ET AL: "A physiologically based model for ethanol and acetaldehyde metabolism in human beings", ALCOHOL, ELSEVIER, AMSTERDAM, NL, vol. 35, no. 1, 1 January 2005 (2005-01-01), pages 3-12, XP004910625, ISSN: 0741-8329, DOI: 10.1016/J.ALCOHOL.2004.11.004
- FUKUWATARI W. & SHIBATA K.: "Consideration of diurnal variations in human blood NAD and DADP concentration.", J. NUTR. SCI. VITAMINOL., vol. 55, 2009, pages 279-281,
- LOPACHIN R.M. & GAVIN T.: "Molecular mechanisms of aldehyde toxicity: a chemical perspective.", CHEM. RES. TOXICOL., vol. 27, 2014, pages 1081-1091,
- HARRISON C.: "Blocking cocaine-seeking behaviour", NATURE REVIEWS (DRUG DISCOVERY), vol. 9, 1 October 2010 (2010-10-01), page 765,

## Description

### CROSS-REFERENCE TO RELATED APPLICATIONS

The present patent claims the priority benefit of U.S. Provisional Patent Application Ser. No. 61/815,206, filed April 23, 2013.

### FIELD OF THE INVENTION

This invention relates to methods and compositions for the treatment of physiological conditions associated with acetaldehyde dehydrogenase 2 (ALDH2) deficiencies.

### BACKGROUND OF THE INVENTION

Alcohol poisoning occurs when a patient drinks a very large amount of alcohol (ethanol) during a short period of time. Very large doses of alcohol cause saturation of the enzymes involved in metabolism of alcohol, thus allowing only a fraction of the alcohol to be converted by metabolic transformation to its non-toxic end product (zero-order kinetics for drug metabolism).

Disulfiram (Antabuse™) is a drug that is used to treat both alcohol and cocaine dependencies. Its mechanism of action is inhibition of the enzyme ALDH2 resulting in accumulation of acetaldehyde and symptoms of acetaldehyde poisoning (or alcohol-disulfiram reaction) after consumption of alcohol. Disulfiram was approved for treatment of alcoholism more than 50 years ago, and was initially used as an implant resulting in 100% sobriety. It also caused multiple deaths from alcohol-disulfiram reaction (acetaldehyde poisoning). To avoid deaths and disulfiram toxicity, disulfiram is now administered orally and dose of the drug decreased from 3,000 mg a day to 250 mg a day. However, this new dosage regime results in reduced overall compliance, as a patient can stop medication and relapse without severe effects. Thus, although current standard of care using oral disulfiram decreases the number of drinking days, it does not ensure abstinence as effectively as an implant (Hughes JC and Cook CC, Addiction 1997 Apr;92(4):381-95). Treatment to reverse the alcohol-disulfiram reaction would allow development of a new generation of long-term disulfiram delivery options without fear of fatalities currently associated with such delivery. The present methods and compositions address this need.

US 5 855 881 A discloses a method of producing mammalian ALDH in plants. US 5 855 881 A further discloses that the ALDH is recovered from the plants and packaged with NAD and a suitable buffer as a pill, paste, or food snack. The composition is taken orally before engaging in social drinking to prevent or ameliorate the effects of alcohol consumption.

A publication of H. Ma et al. (Eur. Heart J. 32(8):1025-1038, 2010) relates to the mechanism involved in mitochondrial aldehyde dehydrogenase induced cardioprotection against ischaemia/reperfusion (I/R) injury with a focus on autophagy.

A publication of H. Ma et al. (J. Am. Coll. Cardiol. 54(23):2187-2196, 2009) relates to the role of facilitated detoxification of acetaldehyde, the main metabolic product of ethanol, through systemic overexpression of mitochondrial aldehyde dehydrogenase-2 (ALDH2) on acute ethanol exposure-induced myocardial damage.

A publication of J. Ren et al. (Toxicol. Lett. 187:149-156, 2009) relates to the impact of facilitated acetaldehyde breakdown via transgenic overexpression of mitochondrial aldehyde dehydrogenase-2 (ALDH2) on alcohol-induced cerebral cortical injury.

Harry (1998; Toxicol. Lett. 95:84) suggests the treatment of disulfiram-ethanol reaction using 4-methylpyrazole.

Chen (2008; Science 321:1493-1495) teaches that Alda-1, a compound that induces ALDH2, may be used to prevent problems before an ischemic event.

### SUMMARY OF THE INVENTION

The invention is defined in the appended claims. In essence, the present invention provides a composition comprising Aldehyde dehydrogenase 2 for use in the treatment of aldehyde toxicity resulting from ethanol metabolism after a subject has consumed ethanol, wherein the composition is to be administered IV in a therapeutically effective dosage, wherein the dosage results in at least a 25% reduction of the systemic level of the toxic aldehyde in the subject.

The present invention further provides a composition comprising Aldehyde dehydrogenase 2 for use in the treatment of aldehyde toxicity resulting from ischemic injury in a subject that has undergone an ischemic event wherein the composition is to be administered IV in a therapeutically effective dosage, wherein the dosage results in at least a 25% reduction of the systemic level of one or more toxic aldehydes selected from 4-hydroxynonenal and malondialdehyde in the subject.

### SUMMARY OF THE TECHNICAL INFORMATION

The technical information set out below may in some respects go beyond the disclosure of the invention per se, which is defined exclusively by the appended claims. The additional technical information is provided to place the actual invention in a broader technical context and to illustrated possible related technical developments. Such additional technical information, which does not fall within the scope of the appended claims, is not part of the invention.

Taught herein are compositions comprising ALDH2 for use in the treatment of a patient with toxicity resulting from ALDH2 deficiency. The physiological states that may be treated using the present invention include temporary ALDH2 deficiency, such as that seen by alcohol poisoning, or genetic ALDH2 deficiency, as is common in certain ethnic populations.

Administration of a composition comprising ALDH2 in a therapeutically effective dosage should result in at least a 25% reduction of the systemic level of toxic chemicals in the subject. More preferably the dosage results in at least a 35% reduction of the systemic level of toxic chemicals in the subject, and even more preferably the dosage results in at least a 50% reduction of the systemic level of toxic chemicals in the subject.

For the purposes of the invention, the chemical is a toxic aldehyde resulting from the metabolism of alcohol; or it is 4-hydroxynonenal and/or malondialdehyde, which are toxic aldehydes resulting from ischemic injury.

The subject treated may have a temporary ALDH2 deficiency, e.g., that caused by a pharmacologically active substance or injury. The subject may also have a permanent genetic ALDH2 deficiency.

ALDH2 or a composition comprising ALDH2 can be administered to a patient exhibiting toxicity due to an acute, temporary ALDH2 deficiency. Commonly, this is caused by ingestion of an amount of a pharmacologically active substance (e.g., alcohol) that overwhelms the natural levels of ALDH2 available for metabolism of that substance. For such patients, this physiological state is temporary, and thus addressing the temporary deficiency may prevent the toxicity otherwise seen and prevent serious organ damage or even death.

Thus, toxicity associated with an ALDH2 deficiency in a subject under the influence of alcohol, can be treated by administering ALDH2 or a composition comprising ALDH2 in an amount sufficient to restore first order kinetics of the pharmacologically active substance. In some cases, the subjects being treated have been administered disulfiram, and thus are more prone to the toxic effects of the pharmacologically active substance due to reduced levels of ALDH2.

Patients undergoing treatment for addiction (*e*.*g*., addiction to alcohol) involving administration of disulfiram may particularly benefit from such methods. The methods can be used as an adjunctive therapy to disulfiram administration, to prevent the potentially lethal effects that may be seen during a relapse. In this context, disulfiram can be delivered using a controlled release dosage format, *e.g.,* an implantable device.

Temporary ALDH2 deficiency may also occur during ischemia-reperfusion injury, resulting in an accumulation of toxic aldehydes and increased size of organ damage. Administration of ALDH2 or a composition comprising ALDH2 following such an injury, and preferably immediately following such an injury, can neutralize the toxic aldehydes and reduce their damaging effects.

Thus, a composition comprising ALDH2 can be administere also to treat toxicity associated with an ALDH2 deficiency in a subject that has undergone an ischemic event, or to treat individuals with permanent ALDH2 deficiency following the consumption of alcohol.

In the above-described methods, preferably, the ALDH2 or composition comprising ALDH2 is administered to a subject in a therapeutically effective dosage from 0.001g per kg body weight to 5g per kg body weight. More preferably ALDH2 or a composition comprising ALDH2 is administered to a subject in a therapeutically effective dosage from 0.1g per kg body weight to 2g per kg body weight. The therapeutically effective dosage will reduce the systemic level of toxic chemicals (e.g., a pharmacologically active substance or a toxic metabolic by-product of a substance) by at least 25% following administration, more preferably by at least 35% reduction following administration, and more preferably by at least 50% reduction measurable within 2-24 hours following administration 12 h.

ALDH2 or a composition comprising ALDH2 can be administered to a patient exhibiting toxicity due to an acute, temporary ALDH2 deficiency. Commonly, this is caused by ingestion of an amount of a pharmacologically active substance (*e.g*., alcohol) that overwhelms the natural levels of ALDH2 available for metabolism of that substance. For such patients, this physiological state is temporary, and thus addressing the temporary deficiency may prevent the toxicity otherwise seen and prevent serious organ damage or even death.

The patient may be undergoing treatment with addiction following ALDH2 deficiency caused by an interaction of the drug disulfiram with alcohol. Administering ALDH2 can be used to prevent or reduce the effects of disulfiram following a relapse.

Thus, ALDH2 administration can be included as part of a comprehensive treatment of addiction treatment that would increase abstinence but not be associated with fatalities, such as in a treatment regime for alcohol addiction, as an adjunct to disulfiram therapy, or to treat acute alcohol poisoning.

To be suitable for administration, the composition will typically comprise ALDH2 and a pharmaceutically suitable carrier. The composition preferably comprises recombinant human ALDH2, and optionally comprises an active agent other than ALDH2 that is useful in treating the condition associated with the ALDH2 deficiency.

One advantage is that administration of ALDH2 or a composition comprising ALDH2 reduces or eliminates the toxicity associated with ALDH2 deficiency in a patient suffering acute temporary ALDH2 deficiency.

A further advantage is that administering ALDH2 can reinstate long-term disulfiram delivery as a very effective treatment option for patients with alcoholism.

### DESCRIPTION OF THE FIGURES

FIG. 1 is a graph showing the effect of drug dose on the rate of metabolism
FIG. 2 is an illustration of ethanol metabolism reactions in the liver.
FIG. 3 is an illustration showing the pathway for toxic aldehyde formation during ischemia-reperfusion injury.
FIG. 4 is a graph illustrating the effects of intraperitoneal ALDH administration following ethanol administration in a rat model.

### DEFINITIONS

The terms used herein are intended to have the plain and ordinary meaning as understood by those of ordinary skill in the art. The following definitions are intended to aid the reader in understanding the present invention, but are not intended to vary or otherwise limit the meaning of such terms unless specifically indicated.

The terms "Aldehyde dehydrogenase" or "ALDH" as used interchangeably herein refers to all or a pharmacologically active form or fragment of any ALDH molecule, including ALDH 1 and ALDH2.

The terms "Aldehyde dehydrogenase 2" or "ALDH2" as used interchangeably herein refers to all or a pharmacologically active form or fragment of the ALDH2 molecule. The ALDH2 may be isolated from a biological source, synthesized or preferably is made using recombinant means. More preferably, the ALDH2 comprises the sequence and structure of human ALDH2. The ALDH2 for use in the present invention may be modified, *e.g.,* to allow tracking of the molecule following administration or to affect the stability of the molecule either before or following administration. The sequence may also be modified to contain certain conserved changes to the protein sequence that allow the molecule to retain its structure and function.

The term "isolated" shall mean separated away from its natural environment. An isolated protein is not necessarily separated away from all materials it is normally present with and may remain associated with certain elements.

The terms "modify", "modification" or "modified" in reference to a protein such as ALDH2 refer to a protein which is altered or derivitized, for example by attachment, linkage, conjugation or complexing with other chemical moieties, by post-translational modification techniques, or by substitution of amino acids.

The terms "PEGylated ALDH2" or "PEG-ALDH2" as used herein refer to an ALDH2 molecule comprising one or more linked PEG molecules.

The terms "treatment", "treating" and "treat" and the like are used herein to generally mean obtaining a desired pharmacological and/or physiological effect. The effect may be prophylactic in terms of completely or partially preventing the toxicity associated with an ALDH2 deficiency or symptom thereof and/or may be therapeutic in terms of partially or completely reducing the adverse effects attributable to ALDH2 deficiency. The "treatment" as used herein covers any treatment of a disease in a mammal, particularly a human.

### DETAILED DISCLOSURE

The technical information set out below may in some respects go beyond the disclosure of the invention per se, which is defined exclusively by the appended claims. The additional technical information is provided to place the actual invention in a broader technical context and to illustrated possible related technical developments. Such additional technical information, which does not fall within the scope of the appended claims, is not part of the invention.

### Purpose

The purpose of the present work is to provide options for the treatment of physiological conditions associated with enzyme deficiencies, and in particular temporary enzyme deficiencies. The use of ALDH2 to battle the effects of a deficiency, whether temporary or permanent, allows the treatment of various physiological conditions and the associated toxicity resulting from such a deficiency.

In the case of alcohol poisoning and alcohol-disulfiram reaction, the administration of ALDH2 or a composition comprising ALDH2 would be a very effective treatment to overcome temporary enzyme deficiency. Successful treatment for alcohol-disulfiram reaction would allow for development of a disulfiram-device combination. Development of this type of device is not possible without undressing safety issues associated with a possibly fatal alcohol-disulfiram reaction.

Temporal ALDH2 enzyme deficiency may also occur as a result of ischemia-reperfusion injury, and administration of ALDH2 or a composition comprising ALDH2 may be beneficial in this patient population as well. The present invention is efficacious in treating events associated with drug use and/or addiction, as the administration of ALDH2 or a composition comprising ALDH2 decreases the toxic level of the drug and end-organ damage associated with it.

ALDH2 enzyme therapy is an effective treatment for alcohol-disulfiram reaction. This reaction occurs when a patient who takes disulfiram consumes alcohol. Disulfiram is non-competitive inhibitor of ALDH2 so it creates an "artificial" deficiency of this enzyme. Treatment of severe alcohol-disulfiram reaction would significantly increase safety of disulfiram. Some other drugs (sulfonylureas, quinacrine, griseofuvin, chloramphenicol and others) can also inhibit this enzyme resulting in disulfiram like reaction when patients drink alcohol. Human recombinant ALDH2 could also be an effective treatment for disulfiram like reaction as well.

### Modification of ALDH2

A major challenge for the therapeutic use of many peptides and proteins is their short circulatory half-life. Thus, in certain embodiments of the invention, it is desirable to modify the ALDH molecule to improve its therapeutic properties. For example, conjugation of a therapeutic protein or peptide sequence with biodegradable polymer can prolong the maintenance of therapeutic drug levels relative to administration of the drug itself. Sustained release may be extended up to several weeks depending on the formulation and the active ingredient conjugated. It will be appreciated that modification as herein described preferably occurs without a substantial loss-of-function or biological activity as compared to the function or biological activity of the unmodified ALDH2 protein.

One preferred chemical moiety for modification of ALDH2 is polyethylene glycol (PEG). PEGylation may significantly improve the physicochemical properties (solubility and stability) of biopharmaceuticals such as ALDH2 while also increasing in vivo circulation half-life, e.g., by decreased enzymatic degradation and/or decreased kidney clearance. PEG conveys to molecules such as proteins its physicochemical properties and therefore modifies also biodistribution and solubility of protein-based biopharmaceuticals. More particularly, PEG conjugation may mask a protein's surface and increase the molecular size of the protein, thus reducing its renal ultrafiltration, preventing the approach of antibodies or antigen processing cells and reducing degradation by proteolytic enzymes.

Modification by PEGylation may occur at random positions or a predetermined position within ALDH2, and may include one, two, three or more attached PEG molecules as described herein. There are several methodologies available for protein PEGylation, and conjugation chemistries. Reference is made to Roberts et al (2002) Advanced Drug Delivery Reviews 54: 459-476 and Zalipsky (1995) Advanced Drug Reviews 16: 157-182, and US Pub No 2011/0196134, which provide non-limiting examples of several methodologies available for protein PEGylation, and conjugation chemistries.

In order to avoid heterogeneous products resulting from random PEGylation, different strategies have been developed for site-specific PEGylation of proteins, by a combination of site-specific mutagenesis and residue-specific chemical reaction. A method is selective thiol PEGylation targeting free cysteine residues. This strategy has been successfully used for site-directed.

PEG may have a branched or unbranched structure. Non-limiting examples of branched PEGs suitable for conjugation to ALDH2 are provided in U.S. Pat. 5,643,575. In particularly preferred embodiments, the PEG is a branched structure.

The PEG molecule suited for use in the present invention may be of any molecular weight between about 1 kDa to about 100 kDa, as practically desired. Typically, although not exclusively, PEG preparations exist as a heterogeneous mixture of PEG molecules either above or below the stated molecular weight. By way of example, the PEG may have an average molecular weight of about 1000, 1500, 2000, 2500, 3000, 3500, 4000, 4500, 5000, 5500, 6000, 6500, 7000, 7500, 8000, 8500, 9000, 9500, 10000, 10500, 11000, 11500, 12000, 12500, 13000, 13500, 14000, 14500, 15000, 15500, 16000, 16500, 17000, 17500, 18000, 18500, 19000, 19500, 20000, 25000, 30000, 35000, 40000, 45000, 50000, 55000, 60000, 65000, 70000, 75000, 80000, 85000, 90000, 95000 or 100000 Da. Preferably, the PEG has an average molecular weight of about between about 4500 Da to about 70000 Da. More preferably, the PEG has an average molecular weight of about 5500 Da.

In another example, conjugated hyaluronic acid can be used to enhance the delivery of ALDH2. HA is a natural linear polysaccharide composed of alternating disaccharide units of D-glucuronic acid and N-acetyl-D-glucosamine with β (1-4) interglycosidic linkage. HA is the only non-sulfated glycosaminoglycan (GAG) which is abundant in synovial fluid and extracellular matrix (ECM). HA with a high molecular weight over 2 million Da was used for the sustained release formulation of human growth hormone (Kim et al, J. Control. Rel. 104, 323-335, 2005) and selectively cross-linked HA hydrogels were used for the encapsulation of erythropoietin (Motokawa et al., J. Biomed. Mater. Res. 78A, 459-465, 2006). HA was also used for the conjugation with active cytotoxic agents, such as paclitaxel (Luo et al, Biomacromolecules 1, 208-218, 2000) and doxorubicin (Luo et al., Pharm. Res. 19, 396-402, 2002).

In another example, the ALDH2 can be modified to be associated with larger proteins to enhance its half-life and/or activity. For example, IgG and albumin both have prolonged half-lives of 19 days compared with few days or less for other circulating proteins.

In yet another example, the structure of ALDH2 can be modified, e.g., via coupling through an amide bond with at least one conformationally rigid substituent, either at the N-terminal of the peptide, the C-terminal of the peptide, on a free amino or carboxyl group along the peptide chain, or at a plurality of these sites (see US Pub No. 2003/0204063) or via substitution of l-analogue amino acids within ALDH2 by d-amino acids.

### Treatment of acute alcohol poisoning

Ethanol poisoning is an acute, life-threatening toxic condition resulting from exposure to excessive quantities of ethanol within a short period of time. Administration of the ALDH2 or a composition comprising ALDH2 can be used for the treatment of alcohol poisoning, as it would help to overcome enzyme saturation and change drug metabolism from zero-order kinetics to first-order kinetics. Under first-order kinetics the amount of a drug which undergoes metabolism is proportional to the drug level rather than constant. This concept is explained in more detail below

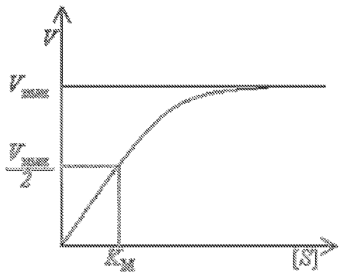

Most metabolic transformations in the body are catalyzed by enzymes and follow Michaelis-Menten kinetics. Under normal conditions, the metabolism of most drugs follows first-order Michaelis-Menten kinetics, and is directly proportional to the drug free blood concentration. However, in the case of an overdose, the metabolism changes from first-order kinetics to zero-order kinetics. Zero-order kinetics occurs because the enzymes are saturated and there are not enough of enzymes to increase drug metabolism. As a result, the amount of the drug that is metabolized over a period of time remains constant and independent from its blood concentration. See Fig. 1, a plot for a dose of ethanol, where V is the rate of drug metabolism, and Kₘ is the Michaelis-Menten constant. At low doses metabolism of alcohol is first order, that is, proportional to drug dose. At high doses, alcohol metabolism is zero-order, constant and independent of concentration.

Ethanol is metabolized in the liver at a rate of approximately one drink per hour and large quantities of alcohol can overload the liver metabolic capacity, causing the blood alcohol concentration (BAL) to rise rapidly. The effects of ethanol on the central nervous system (CNS) are directly proportional to its blood concentration. At high BAL, CNS depression occurs and the heart and lungs become partially anesthetized possibly leading to a coma or death. At BALs of 0.25 to 0.40 g/deciliter, alcohol poisoning can lead to slurred speech, ataxia, stupor, coma and eventually death (Poikolainen K. and Vuori E. Alcohol and Alcoholism 20(3):329-332. 1985).

In the general population, stupor occurs at a BAL of 0.25 to 0.40%. A coma is usually diagnosed at a BAL of 0.35 to 0.50%. All symptoms such as deepening unconsciousness, depressed reflexes, life-threatening respiratory depression and bradycardia become more pronounced at this stage. Death from ethanol consumption is possible when BAL reaches 0.4%. A BAL of 0.5% or more is usually fatal.

Ethanol poisoning occurs as a result of enzyme saturation during metabolism. Treatment with recombinant human aldehyde dehydrogenase 2 (rhALDH2) is proposed to increase the metabolism of both acetaldehyde (a toxic intermediate product) and ethanol.

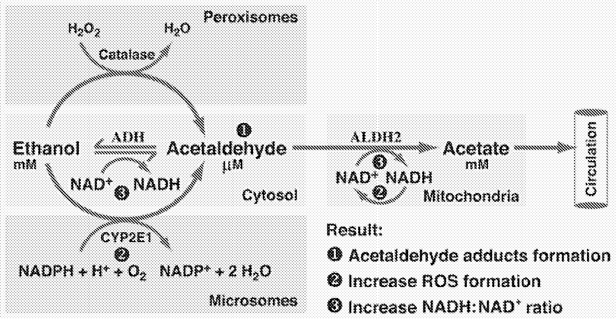

Most ethanol metabolism occurs in the liver cells, as the liver has the highest concentration of enzymes involved in metabolism. In the first reaction of ethanol metabolism, ethanol is oxidized to acetaldehyde via the enzyme alcohol dehydrogenase 1 (ADH1). This first reaction is reversible, and with increased acetaldehyde levels, ethanol and acetaldehyde levels reach equilibrium **(****Fig. 2****).** The same enzyme ADH metabolizes reaction in both directions. Activity of ADH is significantly higher in the reverse reaction, to avoid accumulation of toxic acetaldehyde (Umulis DM et al., Alcohol. 2005 Jan;35(1):3-12.

Under normal conditions, during the second reaction, acetaldehyde, a highly toxic metabolite, is rapidly metabolized by ALDH2 to form acetate. Acetate has significantly less toxic properties compared to acetaldehyde. Acetate is then metabolized in the liver and adipose tissue to form acetyl-CoA. Conversion of acetate to acetyl-CoA is not a rate-limiting reaction. With a high dose of ethanol consumption, both enzymes ADH1 and ALDH2 become saturated, leading to a significant increase of acetaldehyde and ethanol levels in the blood as well as in the liver.

When ALDH2 becomes saturated, the second metabolic reaction (conversion of acetaldehyde to acetate) becomes rate-limiting. After administration of rhALDH2, saturation of ALDH2 is lifted and first order kinetics would be reinstated, driving the reaction to the right. Removal of acetaldehyde would move the equilibrium of the first reaction toward acetaldehyde formation, and as both forward and reverse reaction use the same enzyme there would be a significant increase of amount of the enzyme available for the forward reaction. In result there will be an increase in metabolism of both ethanol and acetaldehyde. Both reactions involve an intermediate carrier of electrons, nicotinamide adenine dinucleotide (NAD⁺), which is reduced by two electrons to form NADH. The concentration of NAD in the blood is sufficient to support increase of the second reaction in the bloodstream. Moving the second reaction from the liver to the blood stream would make more NAD available for the first reaction.

Hence, the metabolism of toxic levels of alcohol and acetaldehyde would be cleared more rapidly to alleviate life-threatening symptoms (coma and death) of CNS depression due to intoxication. Administration of ALDH2 or a composition comprising ALDH2 would allow for the change of ethanol metabolism from zero-order to the first-order and would significantly increase the metabolism of alcohol to its final and non-toxic end product.

### Use of ALDH2 for treatment of drug-disulfiram reaction.

Patients who are taking disulfiram have a deficiency of ALDH2, as it is the mechanism of action for this drug. If a patient takes disulfiram and consumes alcohol they accumulate acetaldehyde and develop symptoms of acetaldehyde poisoning (or alcohol-disulfiram reaction). Fear of this reaction generally prevents people from drinking. However, acetaldehyde is significantly more toxic than ethanol and fatal acetaldehyde poisoning may occur with relatively small doses of ethanol.

Administration of ALDH2 or a composition comprising ALDH2 would help to overcome enzyme deficiency and would allow the acetaldehyde metabolism to occur in the bloodstream rather than liver cells. Exogenous administration of ALDH2 or a composition comprising ALDH2 would be a very effective treatment for alcohol-disulfiram reaction. Treatment with disulfiram becomes much safer, as the potential side effects are no longer unavoidable and likely to be lethal.

Disulfiram may also be used to treat cocaine dependency because disulfiram interferes with the metabolism of cocaine by blocking the enzyme dopamine β-hydroxylase (DBH), and it shows efficacy for this indication as well. The use of ALDH2 or a composition comprising ALDH2 may also limit the toxicity when cocaine is ingested by a person taking disulfiram.

### Use of ALDH2 with controlled release disulfiram

The ability to limit the toxicity observed with a drug-disulfiram reactions allows the use of disulfiram with controlled release dosage formats, such as implantable devices. This enables new treatment regimens and development of a new generation of disulfiram-device combinations which delivers doses that are bioequivalent to orally administered doses used to treat alcoholism.

### Use of ALDH2 in ischemia-reperfusion injury

A significant amount of scientific data shows that mitochondrial ALDH2 enzyme plays a critical role in cardio protection. ALDH2 or a composition comprising ALDH2 can also be used to limit additional an ongoing damage (*e.g.*, remodeling) that takes place in ischemic events by limiting the toxicity associated with toxic aldehydes at the time of the ischemic event.

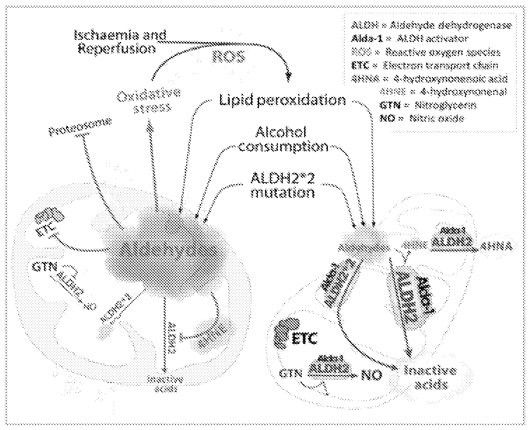

Without being limited by any particular theory, the cardio protective effect of ALDH2 can be explained at least in part by the fact that this enzyme is involved in the detoxification of reactive aldehydes which are created during ischemia-reperfusion injury caused by MI (Budas, G. et al., Trends in Cardiovascular Medicine, Vol. 19, Issue 5, Jul. 2009 p 158-164; Chen C-H et al. Science Vol 321 12 Sep. 2008 p. 1493-5.) (Fig 3). Ischemia-reperfusion creates oxidative stress in myocardial cells and results in excessive production of reactive oxygen species (ROS) and H₂O₂. ROS is produced during both stages of ischemia-reperfusion injury. During the initial ischemic phase, ROS production occurs because of metabolic disarrangement despite low levels of oxygen (Ferrari R. et al., Am. J. Of Clinical Nutrition, 1991;53:21S-22S.). However, when oxygen becomes abundant during reperfusion the process of ROS formation intensifies significantly. (*Id.*)*.* Although ROS are short lived they are highly toxic and reactive molecules and they are causing secondary changes in myocardial cells. Elevated levels of ROS -induced stress lead to lipid peroxidation (oxidative degradation of lipids). ROS creates free radicals that "take" electrons from the cell membranes lipids resulting in cell damage. Lipid peroxidation results in production and accumulation of toxic aldehydes, most important 4- hydroxynonenal (4-HNE) and malondialdehyde (MDA). By virtue of their increased chemical stability, these lipid peroxidation-derived aldehydes diffuse greater distances compared with their precursor ROS and behave as secondary toxic messengers that can propagate and amplify oxidative injury (Srivastava S. et al., Am. J. Physiol Heart Circ Physiol. 283:2612-2619, 2002. First published Aug 22, 2002).

ALDH2 is one of the most important enzymes involved in the metabolism of different aldehydes such as acetaldehyde, 4-HNE and others. ALDH2 metabolizes aldehydes to non-toxic, non reactive acids, which either relatively quickly removed from the body or enter other metabolic pathways. 4-HNE is used as a marker of radical -induced lipid peroxidation (LPO) (Blasig I. et al., Am. J. Physiol., 269 (Heart Circ. Physiol. 38 H 1-H22)). A study performed on the isolated heart of Wistar-Kyoto rats showed statistically significant increases in 4-HNE levels (P < 0.05) 2 min after the onset of reperfusion (after 30 minutes of total and global ischemia) (*Id*). 4-HNE has many negative effects on myocardial cells, and as a cardio-toxin, 4-HNE directly inhibits contractility and induces pro-arrhythmic effects in isolated cardiac myocytes causing additional damage after cardiac ischemia (Chen C-H et al. *Id*.) Use of ALDH2 following an ischemic event can be useful to metabolize 4-HNE and other harmful aldehydes to prevent additional tissue damage.

Inactivation of ALDH2 also reduces the effectiveness of nitroglycerin (GTN), the drug used frequently to treat angina pectoris and MI. GTN main mechanism of action is dilatation of cardiac vessels. ALDH2 mediates bioavailability of GTN by converting it into 1,2-glyceral dinitrate, the active metabolite of the drug. Decreased activity of ALDH2 may be implicated to increase tolerance (decrease response) to GTN in animals as well as humans. Decreased response to GTN may contribute to increased size of MI. GTN itself contributes to decreased activity of ALDH2 resulting in further increase of "aldehydic load" and size of MI (Chen C-H et al. *Id*.)

The toxic effect of endogenous aldehydes not only affect the cells were they are produced, but also adjacent myocardial cells. Aldehydes move easily through cell membranes and may cause adduct formation in other myocardial cells which are not directly affected by severe ischemia. The negative effect of aldehydes on the myocardial cells is well documented. Elevated aldehyde levels from excessive alcohol consumption play a significant role in the development of cardiac alcohol related cardiomyopathy. Environmental and dietary aldehydes may contribute to damage during MI as well (Chen C-H et al. *Id*.)

All these factors implicate toxic, reactive aldehydes in playing a significant role in the pathophysiology of damage caused by acute cardiac ischemia - reperfusion. Mitochondrial ALDH2 plays a significant role in cardio protection by decreasing oxidative stress and by reducing the "aldehydic load" to the myocardial cells (Chen, 2008).

Administration of ALDH2 or a composition comprising ALDH2 during or immediately after the ischemic event can limit the additional damage done by these aldehydes. ALDH2 administration will decrease "aldehydic load" in interstitial fluid, resulting in diffusion of toxic aldehydes from the myocardial cells into interstitial fluid, based on the concentration gradient. It will also decrease aldehyde load from dying myocardial cells to adjacent myocardial cells, and improve survival of cells only partially affected by ischemia.

### Administration

Administration of a compound of the invention is accomplished by intravenous administration. ALDH2 can be incorporated into a variety of formulations for therapeutic administration, including by combining the ALDH2 with appropriate pharmaceutically acceptable carriers. Unit dosage forms for intravenous administration may comprise ALDH2 in a composition as a solution in sterile water, normal saline or another pharmaceutically acceptable carrier. It also may be formulated in a composition with other active agents.

Implants for sustained release formulations are well-known in the art. Implants are formulated as microspheres, slabs, etc. with biodegradable or non-biodegradable polymers. For example, polymers of lactic acid and/or glycolic acid form an erodible polymer that is well-tolerated by the host.

### Dosage

Depending on the patient and condition being treated and on the administration route, the compositions of the invention will generally be administered as a therapeutically effective dosage of from 0.001g per kg body weight to 5g per kg body weight. More preferably, the compositions of the invention will generally be administered as a therapeutically effective dosage of from 0.1g per kg body weight to 2g per kg body weight. Higher doses may be used for localized routes of delivery.

In a subject under the influence of a pharmacologically active substance, the amount of ALDH2 or a composition comprising ALDH2 that is administered is sufficient to result in a measurable decrease in the systemic levels of at least one toxic aldehyde in the subject's bloodstream, and preferably a measurable decrease in two or more toxic aldehydes in the subject's bloodstream. The measurable decrease of toxic aldehydes can is from 5-99%, more preferably the at least a 10% decrease in the systemic levels of toxic aldehydes in the subject's bloodstream, more preferably at least a 20% decrease in the systemic levels of toxic aldehydes in the bloodstream, more preferably at least a 30% decrease in the systemic levels of toxic aldehydes in the bloodstream, more preferably at least a 40% decrease in the systemic levels of toxic aldehydes in the bloodstream, and more preferably at least a 50% decrease in the systemic levels of toxic aldehydes in the bloodstream.

Preferably, in a subject under the influence of a pharmacologically active substance, the amount of ALDH2 or a composition comprising ALDH2 that is administered is sufficient to restore first order kinetics of the pharmacologically active substance.

In a subject that is undergoing or has undergone an ischemic event, the amount of ALDH2 or a composition comprising ALDH2 that is administered is also sufficient to result in a measurable decrease of size of MI or ischemic damage to other organs.

A typical dosage may be: a solution suitable for intravenous administration. Those of skill will readily appreciate that dose levels can vary as the severity of the symptoms and the susceptibility of the subject to side effects. Preferably, the ALDH2 or composition comprising ALDH2 is administered in a level that will return the level of the toxicity to a level not associated with pathology. As this amount will vary according to the amount of a drug ingested or the level of injury sustained, it should be adjusted by the medical practitioner.

### EXAMPLES

The following examples are to be considered as illustrative and not restrictive.

Efforts have been made to ensure accuracy with respect to numbers used (e.g., amounts, temperature, etc.) but some experimental errors and deviations should be accounted for. Unless indicated otherwise, parts are parts by weight, molecular weight is weight average molecular weight, temperature is in degrees centigrade, and pressure is at or near atmospheric.

### Example 1: Intraperitoneal Administration of ALDH

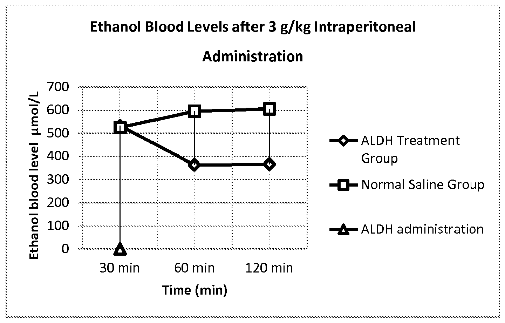

16 Sprague Dawley male rats (weight 275-330 g) were divided into an active treatment group and a placebo group, each group composed of eight animals. Both the active and placebo groups received a 20% ethanol solution, which was administered via intraperitoneal injection. After administration of ethanol, ethanol levels were collected at three time points: Point 0 at 30 minutes following ethanol administration, Point 1 at 1 hour following ethanol administration, and Point 3 at 2 hours following ethanol administration (Fig. 4). The treatment group received the 300 U /saline 200 µL ALDH enzyme solution at Point 0 or 30 minutes after the administration of ethanol. The control group received an equal amount of the normal saline IV at the same time points. One unit of enzyme was defined as the amount of the enzyme that catalyzes the conversion of one micromole of NAD to NADH at pH 8.0 when at room temperature. For each animal the dose of the ALDH enzyme was calculated based on its most recent body weight. As shown in Fig. 4, prior to administration of the enzyme, there was not a statistically significant difference observed between the control and treatments groups. The levels were 533 ± 25.5 and 525 µmol/L ± 31 in treatment and control groups respectively. 30 minutes after administration of the ethanol enzyme (or 1 h after the administration of ethanol) the blood ethanol levels were 363 ± 23.4 in the treatment group and 595 µmol/L ± 16.9 in the control group, which is statistically significant (P< 0.005). 120 minutes after administration of ethanol (point 3) ethanol levels were 366.4 ± 30.5 and 605 µmol/L ± 28.4 also statistically significant. (P< 0. 05))

## Claims

1. A composition comprising Aldehyde dehydrogenase 2 for use in the treatment of aldehyde toxicity resulting from ethanol metabolism after a subject has consumed ethanol, wherein the composition is to be administered IV in a therapeutically effective dosage, wherein the dosage results in at least a 25% reduction of the systemic level of the toxic aldehyde in the subject.

2. Composition for use according to claim 1, **characterized in that** the subject treated has a genetic Aldehyde dehydrogenase 2 deficiency.

3. Composition for use according to claim 1, **characterized in that** the subject treated has a temporary Aldehyde dehydrogenase 2 deficiency.

4. Composition for use according to claim 3, **characterized in that** the subject has been administered disulfiram.

5. Composition for use according to claim 4, **characterized in that** the subject has an alcohol-disulfiram reaction.

6. Composition for use according to claim 3 **characterized in that** subject has been administered one or more selected from sulfonylureas, quinacrine, griseofuvin, and chloramphenicol.

7. Composition for use according to claim 6, **characterized in that** the subject has a disulfiram-like reaction.

8. A composition comprising Aldehyde dehydrogenase 2 for use in the treatment of aldehyde toxicity resulting from ischemic injury in a subject that has undergone an ischemic event wherein the composition is to be administered IV in a therapeutically effective dosage, wherein the dosage results in at least a 25% reduction of the systemic level of one or more toxic aldehydes selected from 4-hydroxynonenal and malondialdehyde in the subject.

9. Composition for use according to anyone of the preceding claims, **characterized in that** the therapeutically effective dosage is from 0.001g per kg body weight to 5g per kg body weight.

10. Composition for use according to anyone of the preceding claims, **characterized in that** the Aldehyde dehydrogenase 2 is PEGylated Aldehyde dehydrogenase.

11. Composition for use according to anyone of the preceding claims, **characterized in that** the composition comprises Aldehyde dehydrogenase 2 and a pharmaceutically suitable carrier.

12. Composition for use according to anyone of the preceding claims, **characterized in that** the composition further contains an additional active agent.

## Patentansprüche

1. Zusammensetzung, umfassend Aldehyddehydrogenase 2 zur Verwendung bei der Behandlung von Aldehydtoxizität, die sich aus dem Ethanolmetabolismus ergibt, nachdem ein Subjekt Ethanol konsumiert hat, wobei die Zusammensetzung IV in einer therapeutisch wirksamen Dosierung zu verabreichen ist, wobei die Dosierung zu einer mindestens 25%-igen Verringerung des systemischen Niveaus des toxischen Aldehyds in dem Subjekt führt.

2. Zusammensetzung zur Verwendung nach Anspruch 1, **dadurch gekennzeichnet, dass** das behandelte Subjekt einen genetischen Aldehyddehydrogenase 2-Mangel aufweist.

3. Zusammensetzung zur Verwendung nach Anspruch 1, **dadurch gekennzeichnet, dass** das behandelte Subjekt einen vorübergehenden Aldehyddehydrogenase 2-Mangel aufweist.

4. Zusammensetzung zur Verwendung nach Anspruch 3, **dadurch gekennzeichnet, dass** dem Subjekt Disulfiram verabreicht wurde.

5. Zusammensetzung zur Verwendung nach Anspruch 4, **dadurch gekennzeichnet, dass** das Subjekt eine Alkohol-Disulfiram-Reaktion aufweist.

6. Zusammensetzung zur Verwendung nach Anspruch 3, **dadurch gekennzeichnet, dass** dem Subjekt eines oder mehrere von Sulfonylharnstoffen, Chinacrin, Griseofuvin und Chloramphenicol verabreicht worden ist.

7. Zusammensetzung zur Verwendung nach Anspruch 6, **dadurch gekennzeichnet, dass** das Subjekt eine Disulfiram-ähnliche Reaktion aufweist.

8. Zusammensetzung, umfassend Aldehyddehydrogenase 2 zur Verwendung bei der Behandlung von Aldehydtoxizität, die sich aus einer ischämischen Verletzung bei einem Subjekt ergibt, das ein ischämisches Ereignis erfahren hat, wobei die Zusammensetzung IV in einer therapeutisch wirksamen Dosierung zu verabreichen ist, wobei die Dosierung zu einer mindestens 25%-igen Verringerung des systemischen Niveaus von einem oder mehreren toxischen Aldehyden, ausgewählt aus 4-Hydroxynonenal und Malondialdehyd, in dem Subjekt führt.

9. Zusammensetzung zur Verwendung nach irgendeinem der vorhergehenden Ansprüche, **dadurch gekennzeichnet, dass** die therapeutisch wirksame Dosierung von 0,001 kg pro kg Körpergewicht bis 5 g pro kg Körpergewicht beträgt.

10. Zusammensetzung zur Verwendung nach irgendeinem der vorhergehenden Ansprüche, **dadurch gekennzeichnet, dass** die Aldehyddehydrogenase 2 PEGylierte Aldehyddehydrogenase ist.

11. Zusammensetzung zur Verwendung nach irgendeinem der vorhergehenden Ansprüche, **dadurch gekennzeichnet, dass** die Zusammensetzung Aldehyddehydrogenase 2 und einen pharmazeutisch geeigneten Träger umfasst.

12. Zusammensetzung zur Verwendung nach irgendeinem der vorhergehenden Ansprüche, **dadurch gekennzeichnet, dass** die Zusammensetzung ferner einen zusätzlichen Wirkstoff enthält.

## Revendications

1. Composition comprenant de l'aldéhyde déshydrogénase 2 pour utilisation dans le traitement d'une toxicité d'aldéhyde résultant d'un métabolisme d'éthanol après qu'un sujet a consommé de l'éthanol, dans laquelle la composition doit être administrée par IV selon un dosage thérapeutiquement efficace, dans laquelle le dosage résulte dans au moins une réduction de 25 % du niveau systémique de l'aldéhyde toxique chez le sujet.

2. Composition pour utilisation selon la revendication 1, **caractérisée en ce que** le sujet traité présente un déficit génétique en aldéhyde déshydrogénase 2.

3. Composition pour utilisation selon la revendication 1, **caractérisée en ce que** le sujet traité présente un déficit temporaire en aldéhyde déshydrogénase 2.

4. Composition pour utilisation selon la revendication 3, **caractérisée en ce que** du disulfirame a été administré au sujet.

5. Composition pour utilisation selon la revendication 4, **caractérisée en ce que** le sujet présente une réaction à l'alcool-disulfirame.

6. Composition pour utilisation selon la revendication 3, **caractérisée en ce qu'**un ou plusieurs sélectionnés parmi les sulfonylurées, la quinacrine, la griséofulvine et le chloramphénicol ont été administrés au sujet.

7. Composition pour utilisation selon la revendication 6, **caractérisée en ce que** le sujet présente une réaction analogue au disulfirame.

8. Composition comprenant de l'aldéhyde déshydrogénase 2 pour utilisation dans le traitement d'une toxicité d'aldéhyde résultant d'une lésion ischémique chez un sujet qui a subi un événement ischémique, dans laquelle la composition doit être administrée par IV selon un dosage thérapeutiquement efficace, dans laquelle le dosage résulte dans au moins une réduction de 25 % du niveau systémique d'un ou plusieurs aldéhydes toxiques sélectionnés parmi le 4-hydroxynonénal et le malondialdéhyde chez le sujet.

9. Composition pour utilisation selon l'une quelconque des revendications précédentes, **caractérisée en ce que** le dosage thérapeutiquement efficace va de 0,001 g par kg de poids corporel à 5 g par kg de poids corporel.

10. Composition pour utilisation selon l'une quelconque des revendications précédentes, **caractérisée en ce que** l'aldéhyde déshydrogénase 2 est de l'aldéhyde déshydrogénase 2 pégylé.

11. Composition pour utilisation selon l'une quelconque des revendications précédentes, **caractérisée en ce que** la composition comprend de l'aldéhyde déshydrogénase 2 et un vecteur pharmaceutiquement approprié.

12. Composition pour utilisation selon l'une quelconque des revendications précédentes, **caractérisée en ce que** la composition contient en outre un agent actif supplémentaire.
